Europäisches Patentamt

European Patent Office

Office européen des brevets

(19)

(11) Veröffentlichungsnummer : **0 209 637**
**B1**

(12) **EUROPÄISCHE PATENTSCHRIFT**

(45) Veröffentlichungstag der Patentschrift :
**30.08.89**

(21) Anmeldenummer : **86104670.4**

(22) Anmeldetag : **05.04.86**

(51) Int. Cl.⁴ : **A 61 F   2/64**

(54) **Brems-Kniegelenk.**

(30) Priorität : **28.05.85 DE 3519046**

(43) Veröffentlichungstag der Anmeldung :
**28.01.87 Patentblatt 87/05**

(45) Bekanntmachung des Hinweises auf die Patenterteilung : **30.08.89 Patentblatt 89/35**

(84) Benannte Vertragsstaaten :
**DE FR GB IT SE**

(56) Entgegenhaltungen :
**AT–B–  243 431**
**DE–C–  661 128**
**DE–C–  843 880**
**DE–U– 8 515 631**
**US–A– 2 400 032**

(73) Patentinhaber : **Otto Bock Orthopädische Industrie Besitz- und Verwaltungs-Kommanditgesellschaft Industriestrasse**
**D-3408 Duderstadt 1 (DE)**

(72) Erfinder : **Haupt, Werner**
**Friedensstrasse 39**
**D-3408 Duderstadt OT Tiftlingerode (DE)**

(74) Vertreter : **Lins, Edgar, Dipl.-Phys. et al Patentanwälte Gramm + Lins Theodor-Heuss-Strasse 2**
**D-3300 Braunschweig (DE)**

EP 0 209 637 B1

## Beschreibung

Die Erfindung betrifft ein Brems-Kniegelenk mit einem Oberschenkelteil und einem Unterschenkelteil, die mit aufeinander angepaßten Bremsflächen versehen sind die bei Belastung aufgrund der relativen Bewegbarkeit des Oberschenkelteils zum Unterschenkelteil gegeneinander gedrückt werden und im wesentlichen eine im Höhenschnitt kreisförmige Kontur· mit einem konstanten Radius ·zur Drehachse aufweisen, und mit einem Anschlag zur Begrenzung der Streckbewegung des Kniegelenkes aus der gebeugten Position.

Ein derartiges Brems-Kniegelenk ist beispielsweise durch die AT-B-243 431 bzw. die US-A-2 400 032 bekannt. Bei Belastung des Kniegelenkes beispielsweise beim Gehen, drücken die Bremsflächen stark aufeinander und bremsen aufgrund der zwischen ihnen bestehenden Reibungskraft die Bewegung des Kniegelenkes. Bei einer vollen Belastung im gestreckten Zustand des Kniegelenkes sorgen die Bremsflächen nahezu für eine Arretierung die erst durch Entlastung des Gelenkes wieder gelöst wird.

Bei einem derartigen Brems-Kniegelenk kann der Unterschenkel relativ zum Oberschenkel bei Entlastung nahezu frei schwingen da die Bremsflächen praktisch keine Reibungskraft erzeugen. Beim Vorbringen des Oberschenkels während des Gehvorganges schwingt der Unterschenkel — unterstützt von einer Vorbringerfeder — nach, bis das Bein gestreckt ist. Diese Vorschwingbewegung des Unterschenkels wird durch einen Anschlag begrenzt. Trotz größerer Bemühungen bei der Konstruktion des Anschlags ist es nicht gelungen einen Anschlag zu erstellen, der für alle Anwendungsfälle bei denen das Vorschwingen des Unterschenkelteils mit mehr oder weniger großem Schwung geschieht zu einer weichen Anschlagbewegung führt. Eine ruckartige, abrupte Beendigung der Vorschwingbewegung war daher bisher nicht zu vermeiden.

Der Erfindung liegt demgegenüber die Aufgabe zugrunde, ein Brems-Kniegelenk der eingangs erwähnten Art zu erstellen bei dem eine ruckartige Beendigung der Vorschwingbewegung des Unterschenkels auch bei stark unterschiedlicher Schwungstärke sicher vermeidbar ist.

Diese Aufgabe wird erfindungsgemäß dadurch gelöst daß die Bremsfläche des Oberschenkelteils ein vorderes Endstück mit einem gegenüber dem konstanten Radius größeren und zunehmenden Abstand zur Drehachse aufweist und daß das vordere Endstück der Bremsfläche des Unterschenkelteils entsprechend angepaßt ist so daß mit zunehmender Streckung des Kniegelenkes im unbelasteten Zustand eine stetig zunehmende Fläche der Bremsfläche in reibenden Kontakt kommt und so den Anschlag bildet.

Erfindungsgemäß wird der Anschlag somit nicht durch ein separates Teil, sondern durch eine geschickte Ausbildung der Bremsflächen gebildet. Durch die erfindungsgemäß ausgestalteten Endstücke der Bremsflächen wird erreicht, daß kurz vor der endgültigen Streckung des Kniegelenkes eine zunehmende Fläche der gegenüber liegenden Bremsfläche aneinander reibt und somit mit einer zunehmenden Bremskraft die Streckbewegung abgebremst wird. Dabei wird ausgenutzt, daß die Bremsflächen in der normalen Funktion des Knies im belasteten Zustand zum Abbremsen geeignet sind, so daß auch eine Eignung zum Abbremsen im unbelasteten Zustand kurz vor dem Erreichen der vollständigen Streckung des Kniegelenkes gegeben ist. Die normale Funktion des Kniegelenkes beim Beugen und wieder Strecken wird durch die Endstücke nicht beeinflußt, weil beim Beugen im Bereich der Endstücke ein deutlicher Spalt zwischen den Bremsflächen entsteht, so daß diese an der Funktion des Brems-Kniegelenkes in diesen Bewegungsstadien nicht teilnehmen.

Das erfindungsgemäße Brems-Kniegelenk läßt sich am zweckmäßigsten realisieren, wenn die Bremsflächen durch senkrecht zur Krümmungsrichtung ebene Streifen gebildet sind. Diese Ausbildung läßt eine gute kosmetische Verkleidung des Kniegelenkes zu, die bei keilförmigen Bremsflächen problematisch ist.

Die Erfindung soll im folgenden anhand eines in der Zeichnung dargestellten Ausführungsbeispiels näher erlautert werden. Es zeigen :

Figur 1 : das erfindungsgemäße Brems-Kniegelenk in einer gebeugten Stellung ;

Figur 2 : das erfindungsgemäße Brems-Kniegelenk in einer Stellung kurz vor der vollständigen Streckung des Kniegelenkes ;

Figur 3 : das erfindungsgemäße Brems-Kniegelenk in seiner vollständigen Streckung.

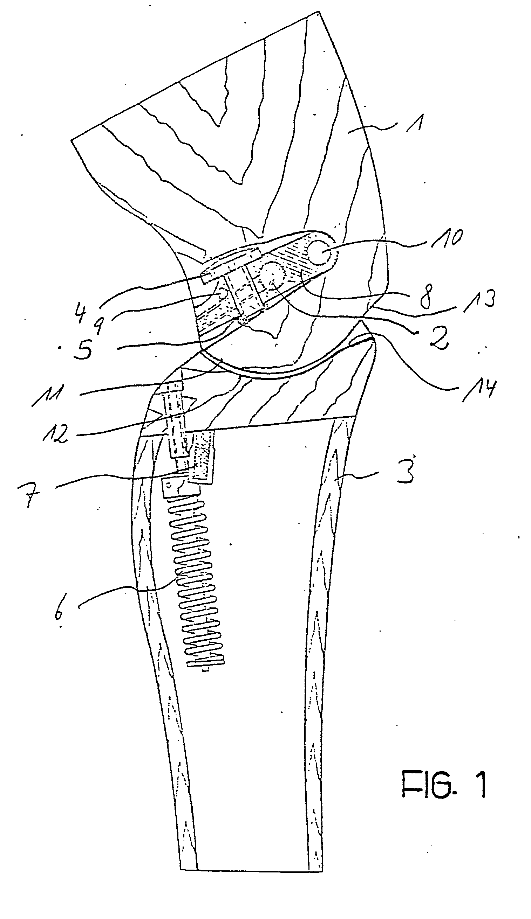

Die Figuren 1 bis 3 zeigen das erfindungsgemäße Brems-Kniegelenk jeweils in einer Höhenschnittdarstellung. Das Kniegelenk besteht aus einem Oberschenkelteil 1 das über eine Drehachse 2 mit einem Unterschenkelteil 3 verbunden ist. Das Unterschenkelteil 3 weist hierzu seitlich hochgebogene Ansätze auf, die bis zur Drehachse 2 das Oberschenkelteil 1 seitlich umschließen, so daß die drehbare Verbindung zwischen Oberschenkelteil 1 und Unterschenkelteil 3 in Höhe der Drehachse 2 erfolgen kann. Das Oberschenkelteil 1 umfaßt die Drehachse 2 mit einem Langloch so daß das Unterschenkelteil 3 relativ zum Oberschenkelteil 1 in der Höhe um ein gewisses Maß bewegbar ist. Diese Höhe ist durch einen Hubversteller 4 einstellbar, in den eine Rückstellfeder 5 integriert ist. Eine im hohlen Unterschenkelteil 3 angeordnete Vorholfeder 6 ist mit Vorhollenkern 7 verbunden, die in einem Schwingblock 8 im Oberschenkelteil 1 an einer Drehachse 9 angelenkt sind. Der Schwingblock 8 ist mittels Bolzen 10 am inneren Ende des Schwingblocks 8 fest mit dem Oberschenkelteil 1 verbunden.

Zur Ausbildung einer Bremsfunktion sind Oberschenkelteil 1 und Unterschenkelteil 3 jeweils mit einer Bremsfläche 11, 12 versehen, die durch ebene Streifen gebildet sind, die im Höhenschnitt im wesentlichen kreisförmig mit einem konstanten Radius zur Drehachse 2 gebogen sind. Am vorderen Ende sind die Bremsflächen 11,12 mit Endstücken 13, 14 versehen, die nach vorn mit einem zunehmenden Abstand zur Drehachse 2 auslaufen.

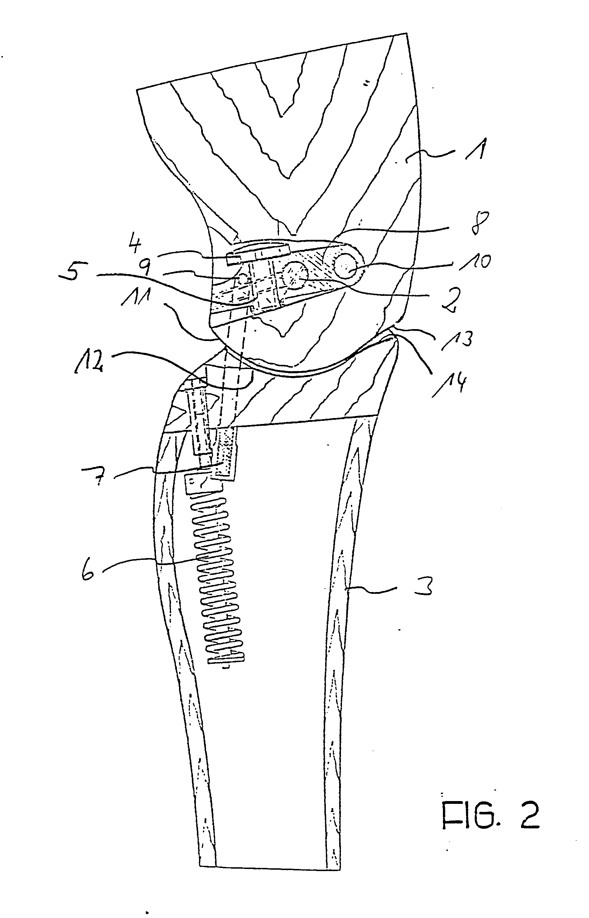

In der in Figur 1 dargestellten, leicht gebeugten Stellung des (entlasteten) Kniegelenks sind die Bremsflächen 11, 12 im Bereich des konstanten Radius zwar in einem leichten Kontakt miteinander, üben jedoch aufgrund ihrer Gleiteigenschaften keine wesentliche Reibungskraft aus. Die Endstücke 13, 14 sind durch einen keilförmigen Zwischenraum voneinander getrennt. Beim Vorschwingen des Unterschenkelteils 3 dreht sich dieses um die Drehachse 2 nach vorn. Figur 2 zeigt eine Stellung, in der die vorderen Endstücke 13, 14 der Bremsflächen 11, 12 gerade im Anschluß an die Teile mit einem konstanten Radius in Kontakt miteinander kommen und dabei eine gewisse Reibungskraft ausüben. Beim weiteren Strecken des Knies vergrößert sich diese gemeinsame Kontaktfläche zwischen den beiden Endstücken 13, 14, so daß der weiteren Drehbewegung des Unterschenkelteils 3 um die Drehachse 2 im Oberschenkelteil 1 eine stetig zunehmende Reibungskraft entgegengesetzt wird, da während der Drehbewegung die Endstücke 13, 14 aufeinander reiben.

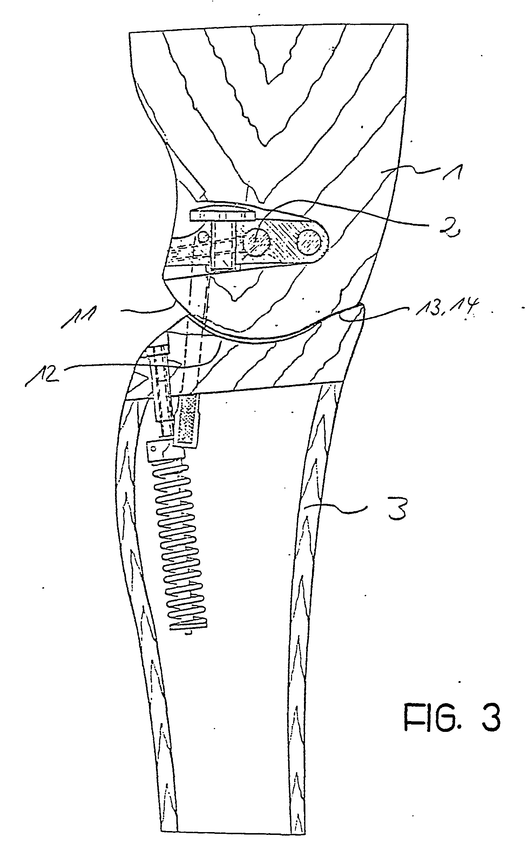

Figur 3 zeigt die gestreckte Endstellung, in der die Endstücke 13, 14 vollständig unter Druck aufeinander liegen ohne daß hierfür eine Belastung des Kniegelenkes bereits erfolgt sei.

Die Endstücke 13, 14 bewirken daher beim Vorschwingen des Unterteils 3 gegenüber dem Oberschenkelteil 1 eine mit zunehmendem Streckungsgrad zunehmende Bremskraft, wodurch die weiche Abbremsung des Unterschenkelteils 3 erzielt wird. Eine weiche Abbremsung wird dabei sowohl bei einem starken Vorwärtsschwung erreicht als auch bei einem schwachen Vorwärtsschwung.

Die Bremsflächen 11, 12 sind vorzugsweise senkrecht zu der Krümmungsrichtung als ebene Streifen ausgebildet. Der Streifen 11 am Oberschenkelteil besteht vorzugsweise aus Vulkanfiber und der Streifen 12 am Unterschenkelteil wird vorzugsweise durch einen silikonbeschichteten Gewebegurt gebildet.

## Patentansprüche

1. Brems-Kniegelenk mit einem Oberschenkelteil (1) und einem Unterschenkelteil (3), die mit aufeinander angepaßten Bremsflächen (11, 12) versehen sind, die bei Belastung aufgrund der relativen Bewegbarkeit des Oberschenkelteils (1) zum Unterschenkelteil (3) gegeneinander gedrückt werden und im wesentlichen eine im Höhenschnitt kreisförmige Kontur mit einem konstanten Radius zur Drehachse (2) aufweisen, und mit einem Anschlag zur Begrenzung der Streckbewegung des Kniegelenkes aus der gebeugten Position dadurch gekennzeichnet, daß die Bremsfläche (11) des oberschenkelteils (1) ein vorderes Endstück (13) mit einem gegenüber dem konstanten Radius größeren und zunehmenden Abstand zur Drehachse (2) aufweist und daß das vordere Endstück (14) der Bremsfläche (12) des Unterschenkelteils (3) entsprechend angepaßt ist, so daß mit zunehmender Streckung des Kniegelenkes im unbelasteten Zustand eine stetig zunehmende Fläche der Bremsflächen (11, 12) in reibenden Kontakt kommt und so den Anschlag bildet.

2. Brems-Kniegelenk nach Anspruch 1, dadurch gekennzeichnet, daß die Bremsflächen (11, 12) durch senkrecht zur Krümmungsrichtung ebene Streifen gebildet sind.

## Claims

1. Braked knee joint with an upper limb part (1) and a lower limb part (3), which are provided with mutually adapted brake surfaces (11, 12) which are pressed together when loaded on account of the movability of the upper limb part (1) relative to the lower limb part (3) and which have in vertical section a substantially circular contour with a constant radius to the axis of rotation (2), and with an abutment for limiting the extension movement of the knee joint from the bent position, characterized in that the brake surface (11) of the upper limb part (1) comprises a forward end portion (13) with a spacing from the axis of rotation (2) which is greater than and increases relative to the constant radius, and in that the forward end portion (14) of the brake surface (12) of the lower limb part (3) is correspondingly adapted, so that with increasing extension of the knee joint in unloaded state there arises a constantly increasing surface of the brake surfaces (11, 12) in frictional contact, thereby forming the abutment.

2. Braked knee joint according to claim 1, characterized in that the brake surfaces (11, 12) are formed by strips flat perpendicular to the direction of curvature.

## Revendications

1. Articulation du genou à effet de freinage, comprenant un élément de jambe supérieure (1) et un élément de jambe inférieure (3) munis de surfaces de freinage adaptées l'une à l'autre qui sont, sous charge, appuyées l'une contre l'autre en raison de la mobilité relative de l'élément de jambe supérieure (1) par rapport à l'élément de jambe inférieure (3) et présentent sensiblement, en coupe en élévation, un contour circulaire à rayon constant par rapport à l'axe de rotation (2), l'articulation comprenant encore une butée limitant le mouvement d'extension de l'articulation à partir de la position pliée, caractérisée en ce que la surface de freinage (11) de l'élément de jambe supérieure (1) comporte une terminaison avant (13) dont la distance à l'axe de rotation (2) est croissante et supérieure au rayon constant, et en ce que la terminaison avant (14) de la surface de freinage (12) de l'élément de jambe inférieure est adaptée de manière correspondante, de façon qu'une surface toujours croissante des surfaces de freinage (11, 12) soit en contact frottant, et constitue ainsi la butée, lorsque l'articulation prend une extension croissante à l'état déchargé.

2. Articulation du genou à effet de freinage, caractérisée en ce que les surfaces de freinage (11, 12) sont constituées par des bandes planes perpendiculaires à la direction de courbure.

FIG. 1

FIG. 2

FIG. 3